(19) Europäisches Patentamt
European Patent Office
Office européen des brevets

(11) Veröffentlichungsnummer: **0 087 561**
**B1**

# (12) EUROPÄISCHE PATENTSCHRIFT

(45) Veröffentlichungstag der Patentschrift: **29.01.86**

(51) Int. Cl.⁴: **C 07 D 231/20**, A 01 N 47/38

(21) Anmeldenummer: **83100338.9**

(22) Anmeldetag: **17.01.83**

(54) **N,N-Dimethyl-0-(1-carbamoyl-5-pyrazolyl)-carbaminsäureester, Verfahren zu ihrer Herstellung und ihre Verwendung als Schädlingsbekämpfungsmittel.**

(30) Priorität: **27.01.82 DE 3202625**

(43) Veröffentlichungstag der Anmeldung:
**07.09.83 Patentblatt 83/36**

(45) Bekanntmachung des Hinweises auf die Patenterteilung:
**29.01.86 Patentblatt 86/5**

(84) Benannte Vertragsstaaten:
**BE CH DE FR GB IT LI NL**

(56) Entgegenhaltungen:
**EP - A - 0 005 240**
**EP - A - 0 009 634**
**EP - A - 0 017 066**
**FR - A - 1 404 687**

(73) Patentinhaber: **BAYER AG, Konzernverwaltung RP Patentabteilung, D-5090 Leverkusen 1 Bayerwerk (DE)**

(72) Erfinder: **Maurer, Fritz, Dr., Roeberstrasse 8, D-5600 Wuppertal 1 (DE)**
Erfinder: **Hammann, Ingeborg, Dr., Lutherstrasse 22, D-4330 Mülheim/Ruhr (DE)**
Erfinder: **Homeyer, Bernhard, Dr., Obere Strasse 28, D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue N,N-Dimethyl-O-(1-carbamoyl-5-pyrazolyl)-carbaminsäureester, ein Verfahren zu ihrer Herstellung und ihre Verwendung als Schädlingsbekämpfungsmittel, insbesondere als Insektizide.

Es ist bekannt, das bestimmte N,N-Dimethyl-O-pyrazolylcarbaminsäureester wie z.B. N,N-Dimethyl-O-(1-phenyl-3-methyl-5-pyrazolyl)- und N,N-Dimethyl-O-(1-isopropyl-3-methyl-5-pyrazolyl)-carbaminsäureester, insektizide Eigenschaften aufweisen (vergleiche CH-PS 279 553). Die insektizide Wirkung dieser bekannten Verbindungen ist jedoch, insbesondere bei niedrigen Wirkstoffkonzentrationen und Aufwandmengen, nicht immer zufriedenstellend.

Es wurden nun neue N,N-Dimethyl-O-(1-carbamoyl-5-pyrazolyl)-carbaminsäureester der Formel I

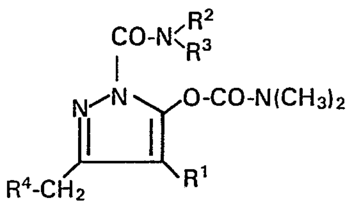

(I)

gefunden, in welcher

$R^1$ für Wasserstoff oder für geradkettiges oder verzweigtes Alkyl mit 1 bis 6 Kohlenstoffatomen steht,

$R^2$ für Wasserstoff oder für geradkettiges oder verzweigtes Alkyl mit 1 bis 6 Kohlenstoffatomen steht,

$R^3$ für Wasserstoff oder geradkettiges oder verzweigtes Alkyl mit 1 bis 6 Kohlenstoffatomen steht, und

$R^4$ für Alkylthio, Alkylsulfinyl oder Alkylsulfonyl, jeweils mit geradkettigem oder verzweigtem Alkylrest mit 1 bis 6 Kohlenstoffatomen steht.

Man erhält sie, wenn man 5-Hydroxy-pyrazole der Formel II

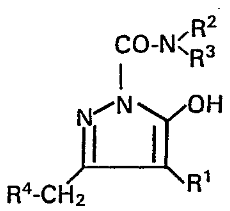

(II)

in welcher

$R^1$, $R^2$, $R^3$ und $R^4$ die oben angegebenen Bedeutungen haben,

oder deren Alkalimetall- bzw. Erdalkalimetallsalze mit N,N-Dimethyl-carbaminsäurehalogeniden der Formel III

$$Hal\text{-}CO\text{-}N(CH_3)_2 \qquad (III)$$

in welcher

Hal für Chlor oder Brom steht,

gegebenenfalls in Gegenwart eines Säureakzeptors und gegebenenfalls unter Verwendung eines inerten Verdünnungsmittels umsetzt.

Die neuen N,N-Dimethyl-O-(1-carbamoyl-5-pyrazolyl)-carbaminsäureester der Formel (I) zeichnen sich durch hohe Wirksamkeit als Schädlingsbekämpfungsmittel, insbesondere durch ihre hervorragende insektizide Wirkung aus.

Überraschenderweise zeigen die erfindungsgemässen N,N-Dimethyl-O-(1-carbamoyl-5-pyrazolyl)-carbaminsäureester eine erheblich höhere insektizide Wirkung als die aus dem Stand der Technik vorbekannten N,N-Dimethyl-O-pyrazolyl-carbaminsäureester wie z.B. N,N-Dimethyl-O-(1-phenyl-3-methyl-5-pyrazolyl)- und N,N-Dimethyl-O-(1-isopropyl-3-methyl-5-pyrazolyl)-carbaminsäureester, welche Verbindungen analoger Konstitution und gleicher Wirkungsrichtung sind.

Aus den Publikationen EP-A 0 017 066 und EP-A 005 240 sind ähnlich strukturierte Pyrazolyl-carbaminsäureester bekannt. Gegenüber diesen Verbindungen zeigen die anmeldungsgemässen Wirkstoffe in überraschendem Masse überlegene biologische Eigenschaften.

Ganz besonders bevorzugt sind die Verbindungen der Formel (I) in welcher

$R^1$ für Wasserstoff, Methyl, Ethyl, n-Propyl oder iso-Propyl steht,

$R^2$ für Wasserstoff, Methyl, Ethyl, n-Propyl, iso-Propyl, n-Butyl oder Iso-Butyl steht,

$R^3$ für Wasserstoff, Methyl, Ethyl, n-Propyl, iso-Propyl, n-Butyl oder iso-Butyl steht, und

$R^4$ für Methoxy, Ethoxy, n-Propoxy, iso-Propoxy, n-Butoxy, iso-Butoxy, sec-Butoxy, Methylthio, Ethylthio, n-Propylthio, iso-Propylthio, n-Butylthio, iso-Butylthio, sec-Butylthio, Methylsulfinyl, Ethylsulfinyl, n-Propylsulfinyl, iso-Propylsulfinyl, Methylsulfonyl, Ethylsulfonyl, n-Propylsulfonyl und iso-Propylsulfonyl steht.

Verwendet man beispielsweise für das erfindungsgemässe Verfahren als Ausgangsstoffe 1-Diethylcarbamoyl-3-methoxymethyl-5-hydroxypyrazol oder das entsprechende Natriumsalz und Dimethylcarbamoylchlorid, so können die entsprechenden Reaktionen durch folgende Formelschemata skizziert werden:

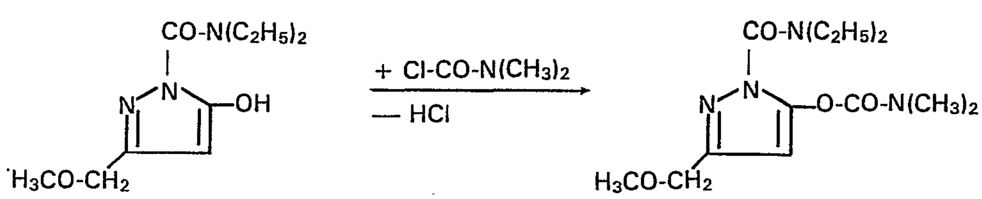

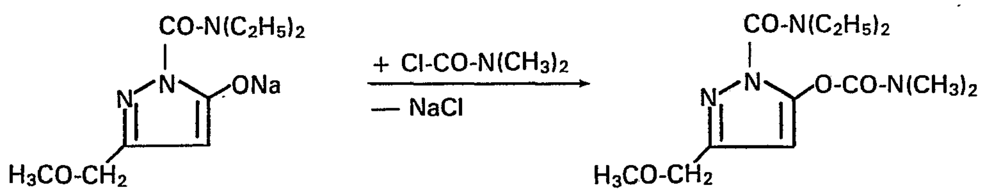


Die bei dem erfindungsgemässen Verfahren als Ausgangsstoffe zu verwendenden 5-Hydroxypyrazole bzw. deren Alkali- und Erdalkalisalze sind durch Formel (II) definiert. Vorzugsweise stehen darin $R^1$, $R^2$, $R^3$ und $R^4$ für diejenigen Reste, welche bei der Definition der Reste $R^1$, $R^2$, $R^3$ und $R^4$ in Formel (I) als bevorzugt genannt wurden. Die Alkali- und Erdalkalimetallionen stehen vorzugsweise für Natrium-, Kalium-, Calcium- und Ammoniumionen.

Die 5-Hydroxypyrazole bzw. ihre Alkali- und Erdalkalimetallsalze können nach im Prinzip bekannten Verfahren hergestellt werden (vergleiche DE-OS 2 644 588). Man erhält sie beispielsweise durch Umsetzung von entsprechenden Acetessigsäurealkylester-Derivaten mit Hydrazinderivaten $H_2N$-NH-CO-N($R^2$)($R^3$) ($R^2$ und $R^3$ haben die oben angegebenen Bedeutungen) bei Temperaturen zwischen 0 und 100°C, vorzugsweise zwischen 20 und 80°C, gegebenenfalls unter Verwendung von Alkali- bzw. Erdalkalimetallsalzen wie z.B. Natriumsulfat und gegebenenfalls unter Verwendung eines Lösungsmittels wie z.B. Toluol (siehe Herstellungsbeispiele).

Die als Vorprodukte für die Herstellung der neuen Verbindungen der Formel (I) einzusetzenden Acetessigsäurealkylester-Derivate sind bekannt (vergleiche z.B. DE-OS 2 644 588 und DE-OS 2 912 494).

Die als Vorprodukte für die Herstellung der neuen Verbindungen der Formel (I) einzusetzenden Hydrazin-Derivate sind allgemein bekannte Verbindungen der organischen Chemie.

Als Beispiele für die Ausgangsstoffe der Formel (II) seien genannt:

3-Methoxy-methyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-carbamoyl-5-hydroxy-pyrazol;

3-Methoxymethyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-1-(di)-methyl-carbamoyl-5-hydroxy-pyrazol;

3-Methoxymethyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(di)ethylcarbamoyl-5-hydroxy-pyrazol;

3-Methoxymethyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(di)-n-propylcarbamoyl-5-hydroxy-pyrazol;

3-Methoxymethyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(di)-iso-propylcarbamoyl-5-hydroxy-pyrazol;

3-Methoxymethyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(methylethylcarbamoyl)-5-hydroxy-pyrazol;

3-Methoxy-methyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(methyl-n-propylcarbamoyl)-5-hydroxy-pyrazol;

3-Methoxy-methyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(methyl-n-butylcarbamoyl-5-hydroxy-pyrazol;

3-Methoxy-methyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(methyl-iso-butylcarbamoyl-5-hydroxy-pyrazol;

3-Methoxy-methyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(ethyl-n-propylcarbamoyl)-5-hydroxy-pyrazol;

3-Methoxymethyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(ethyl-iso-propylcarbamoyl)-5-hydroxy-pyrazol;

3-Methoxymethyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(ethyl-n-butylcarbamoyl)-5-hydroxy-pyrazol;

3-Methoxymethyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(ethyl-iso-butylcarbamoyl)-5-hydroxy-pyrazol;

3-Methoxymethyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(n-propyl-isopropylcarbamoyl)-5-hydroxy-pyrazol;

3-Methoxy-methyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(n-propyl-n-butylcarbamoyl)-5-hydroxy-pyrazol;

3-Methoxymethyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(n-propyl-iso-butylcarbamoyl)-5-hydroxy-pyrazol;

3-Methoxy-methyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(iso-propyl-n-butylcarbamoyl-5-hydroxy-pyrazol;

3-Methoxy-methyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(iso-propyl-iso-butylcarbamoyl-5-hydroxy-pyrazol;

3-Methoxy-methyl-, 3-Ethoxymethyl-, 3-n-Propoxymethyl-, 3-iso-Propoxymethyl-, 3-n-Butoxymethyl-, 3-iso-Butoxymethyl-, 3-sec-Butoxymethyl-, 3-Methylthiomethyl-, 3-Ethylthiomethyl-, 3-n-Propylthiomethyl-, 3-iso-Propylthiomethyl-, 3-n-Butylthiomethyl-, 3-iso-Butylthiomethyl-, 3-sec-Butylthiomethyl-, 3-Methylsulfinylmethyl-, 3-Ethylsulfinylmethyl-, 3-n-Propylsulfinylmethyl-, 3-iso-Propylsulfinylmethyl-, 3-Methylsulfonylmethyl-, 3-Ethylsulfonylmethyl-, 3-n-Propylsulfonylmethyl- und 3-iso-Propylsulfonylmethyl-4-methyl-, 4-ethyl-, -4-n-propyl- und -4-iso-propyl-1-(n-butyl-iso-butylcarbamoyl-5-hydroxy-pyrazol;

und die entsprechenden Natrium-, Kalium-, Calcium- und Ammoniumsalze.

Als Beispiel für die zu verwendenden Carbaminsäurehalogenide der Formel (III) sei N,N-Dimethylcarbaminsäurechlorid genannt. N,N-Dimethylcarbaminsäurechlorid ist eine allgemein bekannte Verbindung der organischen Chemie.

Das erfindungsgemässe Verfahren zur Herstellung der neuen N,N-Dimethyl-O-(1-carbamoyl-5-pyrazol)-carbaminsäureester wird bevorzugt unter Verwendung von Verdünnungsmitteln durchgeführt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether, und Diglycoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z.B. Acetonitril und Propionitril, Amide, wie z.B. Dimethylformamid, Dimethylacetamid und N-Methylpyrrolidon, sowie Dimethylsulfoxid, Tetramethylensulfon und Hexamethylphosphorsäuretriamid.

Das Verfahren kann gegebenenfalls in Gegenwart von Säureakzeptoren durchgeführt werden. Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Das erfindungsgemässe Verfahren wird im allgemeinen bei Temperaturen zwischen 0 und 100°C durchgeführt. Bevorzugt wird der Bereich zwischen 20 und 80°C. Die Umsetzungen werden im allgemeinen bei Normaldruck durchgeführt. Zur Durchführung des erfindungsgemässen Verfahrens werden die Ausgangsstoffe gewöhnlich in äquimolaren Mengen eingesetzt. Ein Überschuss der einen oder anderen Reaktionskomponente bringt keine wesentlichen Vorteile. Die Umsetzung wird im allgemeinen in einem geeigneten Verdünnungsmittel und gegebenenfalls in Gegenwart eines Säureakzeptors durchgeführt. Nach Reaktionsende giesst man die Mischung in Wasser und schüttelt mit einem organischen Lösungsmittel, z.B. Toluol aus. Die organische Phase wird dann wie üblich durch Waschen, Trocken und Abdestillieren des Lösungsmittels aufgearbeitet.

Die neuen Verbindungen fallen zum Teil in Form von Ölen an, die sich zum Teil nicht unzersetzt destillieren lassen, jedoch durch sogenanntes 'Andestillieren', d.h. durch längeres Erhitzen unter vermindertem Druck auf mässig erhöhte Temperaturen von den letzten flüchtigen Anteilen befreit und auf diese Weise gereinigt werden. Zu ihrer Charakterisierung dient der Brechungsindex.

Soweit die neuen Produkte nach dem Abdestillieren des Lösungsmittels in fester Form anfallen, werden sie durch Umkristallisation gereinigt. Zur Charakterisierung dient der Schmelzpunkt.

Die Wirkstoffe eignen sich bei guter Pflanzenverträglichkeit und günstiger Warmblütertoxizität zur Bekämpfung von tierischen Schädlingen, insbesondere Insekten, und Spinnentieren die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.b. Reticulitermes spp.

Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der der Homoptera z.B. Aleurodes brassicae Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Christoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomya spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp.
Aus der Ordnung der Arachnida z.B. Scoprio maurus, Latrodectus mactans.
Aus der Ordnung der Acarina z.B. Acarus siro, Argas spp., Ornithodoros spp., Dermanysssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykoläther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemässen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

*Beispiel A*

Grenzkonzentrations-Test / Wurzelsystemische Wirkung

| | |
|---|---|
| Testinsekt: | Myzus persicae |
| Lösungsmittel: | 3 Gewichtsteile Aceton |
| Emulgator: | 1 Gewichtsteil Alkylarylpolyglykoläther |

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschliesslich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: (1), (2), (3) und (9).

*Beispiel B*

Myzus-Test

| | |
|---|---|
| Lösungsmittel: | 3 Gewichtsteile Dimethylformamid |
| Emulgator: | 1 Gewichtsteil Alkylarylpolyglykoläther |

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, dass alle Blattläuse abgetötet wurden; 0% bedeutet, dass keine Blattläuse abgetötet wurden.

Bei diesem Test zeigen z.B. die folgenden Verbindungen der Herstellungsbeispiele überlegene Wirksamkeit gegenüber dem Stand der Technik: (1), (2) und (3).

*Herstellungsbeispiele*

*Beispiel 1*

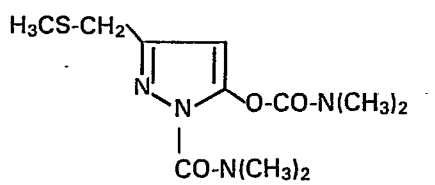

Eine Mischung aus 14,2 g (0,06 Mol) 1-Dimethyl--carbamoyl-3-methyl-thiomethyl-5-hydroxy-pyrazol-Natriumsalz, 200 ml Acetonitril und 6,5 g (0,06 Mol) Dimethylcarbamoylchlorid wird 3 Stunden bei 55-60 °C gerührt. Dann wird das Reaktionsgemisch auf 20 °C abgekühlt und vom anorganischen Salz abgesaugt. Das Filtrat wird im Vakuum eingedampft. Zurück bleiben 14,3 g (83% der Theorie) N,N-Dimethyl-O-(1-dimethylcarbamoyl-3-methylthiomethyl--5-pyrazolyl)-carbamin-Säureester in Form beiger Kristalle mit dem Schmelzpunkt 36°C.

In analoger Weise können die folgenden Verbindungen der Formel (I)

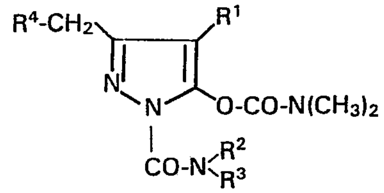

hergestellt werden:

| Beispiel Nr. | $R^1$ | $R^2$ | $R^3$ | $R^4$ | Ausbeute (% der Theorie) | Schmelzpunkt (°C); Brechungsindex; |
|---|---|---|---|---|---|---|
| 2 | H | $CH_3$ | $CH_3$ | $CH_3SO$ | 72 | 88 |
| 3 | H | $CH_3$ | Ch3 | $CH_3SO_2$ | 77 | 118 |
| 4 | H | $CH_3$ | $CH_3$ | $C_2H_5S$ | | |
| 5 | $CH_3$ | $CH_3$ | $CH_3$ | $CH_3S$ | | |
| 6 | $CH_3$ | $CH_3$ | $CH_3$ | $CH_3SO$ | | |
| 7 | $CH_3$ | $CH_3$ | $CH_3$ | $CH_3$-$SO_2$ | | |
| 8 | H | Ch3 | H | $CH_3S$ | 30 | zähes Öl |
| 9 | H | H | H | $CH_3S$ | 72 | $N_D^{20}$: 1,5410 |
| 10 | H | H | $C_2H_5$ | $CH_3S$ | | |
| 11 | H | $CH_3$ | $CH_3$ | n-$C_3H_7S$ | | |
| 12 | H | $CH_3$ | $CH_3$ | $CH_3O$ | | |
| 13 | H | $C_2H_5$ | $C_2H_5$ | $CH_3S$ | | |

Die als Vorprodukte zu verwendenden 5-Hydroxy--pyrazole bzw. deren Alkali- oder Erdalkalisalze können z.B. wie folgt hergestellt werden:

*Beispiel a*

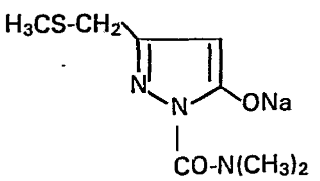

Eine Lösung von 10,3 g (0,1 Mol) 4,4-Dimethylsemicarbazid in 50 ml Toluol wird bei 30-35°C mit 17,6 g (0,1 Mol) 4-Methylthioacet-essigsäure-ethylester versetzt. Nach 4 Stunden gibt man 8 g Natriumsulfat zu, filtriert und versetzt das Filtrat mit 5,4 g (0,1 Mol) festem Natriummethylat. Die Temperatur des Gemisches wird dabei auf 20 bis 25°C gehalten. Nach 1 Stunde gibt man 200 ml Ether zu und saugt das Produkt nach Kristallisation ab. Man erhält so 17 g (72% der Theorie) 1-Dimethylcarbamoyl-3-methylthiomethyl-5-hydroxy-pyrazol-Natriumsalz mit dem Schmelzpunkt 164°C*.

In analoger Weise können die folgenden Verbindungen der Formel (IIa)

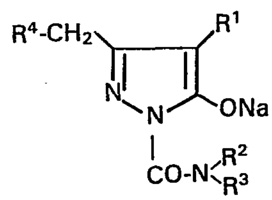

(IIa)

hergestellt werden:

| Beispiel Nr. | $R^1$ | $R^2$ | $R^3$ | $R^4$ | Ausbeute (% der Theorie) | Physikal. Konstante (Brechungsindex); Schmnelzpunkt [°C] |
|---|---|---|---|---|---|---|
| b | H | $CH_3$ | $CH_3$ | $C_2H_5S$ | | |
| c | $CH_3$ | $CH_3$ | $CH_3$ | $CH_3S$ | | |
| d | H | $CH_3$ | H | $CH_3S$ | 92 | 132* |
| e | H | H | H | $CH_3S$ | 80 | 145* |
| f | H | H | $C_2H_5$ | $CH_3S$ | | |
| g | H | $CH_3$ | $CH_3$ | n-$C_3H_7S$ | | |
| h | H | $CH_3$ | $CH_3$ | $CH_3O$ | | |
| i | H | $C_2H_5$ | $C_2H_5$ | $CH_3S$ | | |

* Die Physikalischen Konstanten sind angegeben für die freien Hydroxypyrazole, die man durch Ansäuern der wässrigen Lösung der Natrium-Salze erhalten kann.

**Patentansprüche**

1. N,N-Dimethyl-O-(1-carbamoyl-5-pyrazolyl)-carbaminsäureester der Formel I

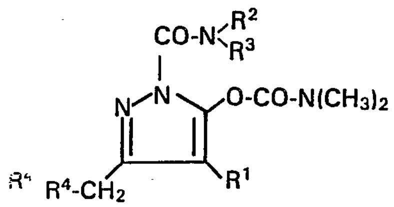

(I)

in welcher

$R^1$ für Wasserstoff oder für geradkettiges oder verzweigtes Alkyl mit 1 bis 6 Kohlenstoffatomen steht,

$R^2$ für Wasserstoff oder für geradkettiges oder verzweigtes Alkyl mit 1 bis 6 Kohlenstoffatomen steht,

$R^3$ für Wasserstoff oder geradkettiges oder verzweigtes Alkyl mit 1 bis 6 Kohlenstoffatomen steht, und

$R^4$ für Alkylthio, Alkylsulfinyl oder Alkylsulfonyl, jeweils mit geradkettigem oder verzweigtem Alkylrest mit 1 bis 6 Kohlenstoffatomen steht.

2. Verfahren zur Herstellung der N,N-Dimethyl-O-(1-carbamoyl-5-pyrazolyl)-carbaminsäureester der Formel (I),

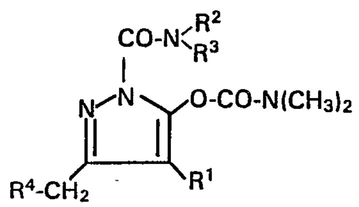

(I)

in welcher $R^1$, $R^2$, $R^3$ und $R^4$ die in Anspruch 1 angegebene Bedeutung besitzen,
dadurch gekennzeichnet, dass man 5-Hydroxy-pyrazole der Formel II

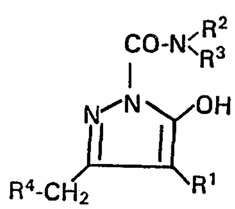

(II)

in welcher
$R^1$, $R^2$, $R^3$ und $R^4$ die in Anspruch 1 angegebene Bedeutung haben,
oder deren Alkalimetall- bzw. Erdalkalimetallsalze mit N,N-Dimethyl-carbaminsäurehalogeniden der Formel III

$$Hal\text{-}CO\text{-}N(CH_3)_2 \qquad (III)$$

in welcher
Hal für Chlor oder Brom steht,
gegebenenfalls in Gegenwart eines Säureakzeptors und gegebenenfalls unter Verwendung eines inerten Verdünnungsmittels umsetzt.

3. Verbindungen gemäss Anspruch 1 der Formel (I) in welcher

$R^1$ für Wasserstoff oder Methyl steht,

$R^2$ für Alkyl mit 1 bis 6 Kohlenstoffatomen steht,

$R^3$ für Alkyl mit 1 bis 6 Kohlenstoffatomen steht,

$R^4$ für Methylthio, Methylsulfinyl oder Methylsulfonyl steht.

4. Schädlingsbekämpfungsmittel, gekennzeichnet durch einen Gehalt an mindestens einem N,N-Dimethyl-O-(1-carbamoyl-5-pyrazolyl)-carbaminsäureester der Formel (I) gemäss Anspruch 1.

5. Verwendung von N,N-Dimethyl-O-(1-carbamoyl-5-pyrazolyl)-carbaminsäureester der Formel (I) gemäss Anspruch 1 zur Bekämpfung von Schädlingen.

6. Verfahren zur Bekämpfung von Schädlingen, dadurch gekennzeichnet, dass man N,N-Dimethyl-O-(1-carbamoyl-5-pyrazolyl)-carbaminsäureester der Formel (I) gemäss Anspruch 1 auf Schädlinge und/oder ihren Lebensraum einwirken lässt.

7. Verfahren zur Herstellung von Schädlingsbekämpfungsmitteln, dadurch gekennzeichnet, dass man N,N-Dimethyl-O-(1-carbamoyl-5-pyrazolyl)-carbaminsäureester der Formel (I) gemäss Anspruch 1 mit Streckmitteln und/oder oberflächenaktiven Mitteln vermischt.

**Claims**

1. N,N-dimethyl-O-(1-carbamoyl-5-pyrazolyl)-carbamic acid esters of the formula I

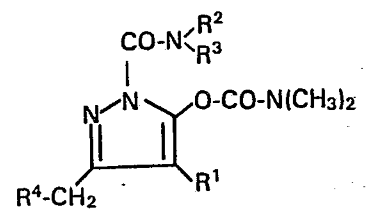

(I)

in which

$R^1$ represents hydrogen or straight-chain or branched alkyl with 1 to 6 carbon atoms,

$R^2$ represents hydrogen or straight-chain or branched alkyl with 1 to 6 carbon atoms,

$R^3$ represents hydrogen or straight-chain or branched alkyl with 1 to 6 carbon atoms,

$R^4$ represents alkyltio, alkylsulphinyl or alkylsulphonyl, each with a straight-chain or branched alkyl radical with 1 to 6 carbon atoms.

2. Process for the preparation of the N,N-dimethyl-O-(1-carbamoyl-5-pyrazolyl)-carbamic acid esters of the formula (I)

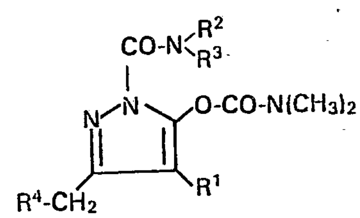

(I)

in which
$R^1$, $R^2$, $R^3$ and $R^4$ have the meaning given in claim 1, characterised in that 5-hydroxy-pyrazoles of the formula II

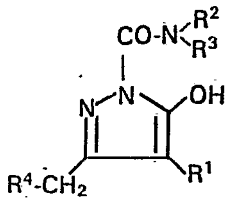

(II)

in which
$R^1$, $R^2$, $R^3$ and $R^4$ have the meaning given in claim 1, or alkali metal salts or alkaline earth metal salts thereof are reacted with N,N-dimethyl-carbamic acid halides of the formula III

$$Hal\text{-}CO\text{-}N(CH_3)_2 \qquad (III)$$

in which
Hal represents chlorine or bromine,
if appropriate in the presence of an acid acceptor and if appropriate using an inert diluent.

3. Compounds according to claim 1 of the formula (I) in which
$R^1$ represents hydrogen or methyl,
$R^2$ represents alkyl with 1 to 6 carbon atoms,
$R^3$ represents alkyl with 1 to 6 carbon atoms, and
$R^4$ represents methylthio, methylsulphinyl or methylsulphonyl.

4. Agents for combating pests, characterised in that they contain at least one N,N-dimethyl-O-(1-carbamoyl-5-pyrazolyl)-carbamic acid ester of the formula (I) according to claim 1.

5. Use of N,N-dimethyl-O-(1-carbamoyl-5-pyrazolyl)-carbamic acid ester of the formula (I) according to claim 1 for combating pests.

6. Process for combating pests, characterised in that N,N-dimethyl-O-(1-carbamoyl-5-pyrazolyl)-carbamic acid esters of the formula (I) according to claim 1 are allowed to act on pests and/or their environment.

7. Process for the preparation of agents for combating pests, characterised in that N,N-dimethyl-O-(1-carbamoyl-5-pyrazolyl)-carbamic acid esters of the formula (I) according to claim 1 are mixed with extenders and/or surface-active agents.

**Revendications**

1. Esters de O-(1-carbamoyl-5-pyrazolyle) d'acide N,N-diméthylcarbamique de formule I

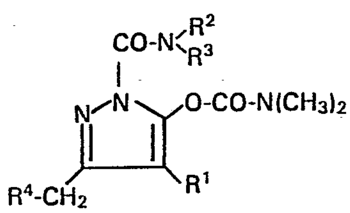

(I)

dans laquelle
$R^1$ représente l'hydrogène ou un groupe alkyle à chaîne droite ou ramifié ayant 1 à 6 atomes de carbone,
$R^2$ est l'hydrogène ou un groupe alkyle à chaîne droite ou ramifié ayant 1 à 6 atomes de arbone,
$R^3$ représente l'hydrogène ou un groupe alkyle à chaîne droite ou ramifié ayant 1 à 6 atomes de carbone, et
$R^4$ est un groupe alkylthio, alkylsulfinyle ou alkylsulfonyle chacun ayant un reste alkyle chaîne droite ou ramifié ayant 1 à 6 atomes de carbone.

2. Procédé de production d'esters de O-(1-carbamoyl-5-pyrazolyle) d'acide N,N-diméthylcarbamique de formule (I)

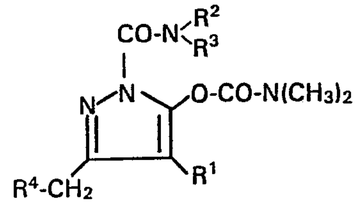

(I)

dans laquelle $R^1$, $R^2$, $R^3$ et $R^4$ ont la définition donnée dans la revendication 1,
caractérisé en ce qu'on fait réagir des 5-hydroxypyrazoles de formule II

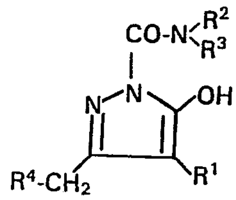

(II)

dans laquelle
$R^1$, $R^2$, $R^3$ et $R^4$ ont la définition donnée dans la revendication 1, ou leurs sels de métaux alcalins ou de métaux alcalino-terreux avec des halogénures d'acide N,N-diméthylcarbamique de formule III

$$Hal\text{-}CO\text{-}N(CH_3)_2 \qquad (III)$$

dans laquelle
Hal représente le chlore ou le brome,
éventuellement en présence d'un accepteur d'acide et, le cas échéant, en utilisant un diluant inerte.

3. Composés suivant la revendication 1, de formule (I) dans laquelle
$R^1$ représente l'hydrogène ou le groupe méthyle,
$R^2$ est un groupe alkyle ayant 1 à 6 atomes de carbone,
$R^3$ est un groupe alkyle ayant 1 à 6 atomes de carbone,
$R^4$ est un groupe méthylthio, méthylsulfinyle ou méthylsulfonyle.

4. Compositions pesticides, caractérisées par une teneur en au moins un ester de O-(1-carbamoyl-5-pyrazolyle) d'acide N,N-diméthylcarbamique de formule (I) suivant la revendication 1.

5. Utilisation d'esters de O-(1-carbamoyl-5-pyrazolyle) d'acide N,N-diméthylcarbamique de formule (I) suivant la revendication 1 pour combattre des parasites.

6. Procédé pour combattre des parasites, caractérisé en ce qu'on fait agir des esters de O-(1-carbamoyl-5-pyrazolyle) d'acide N,N-diméthylcarbamique de formule (I) suivant la revendication 1 sur les parasites et/ou sur leur habitat.

7. Procédé de préparation de compositions pesticides, caractérisé en ce qu'on mélanges des esters de O-(1-carbamoyl-5-pyrazolyle) d'acide N,N-diméthylcarbamique de formule (I) suivant la revendication 1 avec des diluants et/ou des agents tensioactifs.